# EUROPÄISCHE PATENTSCHRIFT

(11) **EP 0 885 013 B1**
(45) Veröffentlichungstag und Bekanntmachung des Hinweises auf die Patenterteilung: **01.08.2001**
(21) Anmeldenummer: 97922838.4
(22) Anmeldetag: 14.04.1997
(51) Int. Cl.: A61K 39/275

(54) **VERWENDUNG VON MULTIPOTENTEN PARAMUNITÄTSINDUCERN AUS ATTENUIERTEN, NICHT-IMMUNOGENEN POCKENVIREN ODER PARAPOCKENVIREN ZUR HERSTELLUNG VON ARZNEIMITTELN**
USE OF MULTIPOTENT PARAPOX IMMUNITY INDUCERS FROM ATTENUATED, NON-IMMUNOGENIC POX VIRUSES OR PARAPOX VIRUSES FOR THE PREPARATION OF MEDICAMENTS
UTILISATION D'INDUCTEURS PARA-IMMUNITAIRES OBTENUS A PARTIR DE POXVIRUS ET PARAPOXVIRUS ATTENUES NON IMMUNOGENES POUR LA PREPARATION DE MEDICAMENTS

(30) Priorität: 15.04.1996 DE 19614810
(43) Veröffentlichungstag der Anmeldung: 23.12.1998
(73) Patentinhaber: Mayr, Anton, Prof. Dr.med.vet. Dr.h.c.mult., 82319 Starnberg (DE)
(72) Erfinder: Mayr, Anton, Prof. Dr.med.vet. Dr.h.c.mult., 82319 Starnberg (DE)
(74) Vertreter: Vossius, Volker, Dr.
(86) Internationale Anmeldenummer: DE9700761
(87) Internationale Veröffentlichungsnummer: WO9738724

(56) Entgegenhaltungen:
- DE-A- 3 504 940
- DE-C- 4 405 841
- TIERÄRZTL. PRAX., Bd. 23, 1995, Seiten 542-552, XP002039312 MAYR B. UND MAYR A.: "ZUM DERZEITIGEN STAND DER PRÄKLINISCHEN FORSCHUNG ÜBER DIE WIRKSAMKEIT UND UNSCHÄDLICHKEIT VON PARAMUNITÄTSINDUCERN AUS POCKENVIREN" in der Anmeldung erwähnt
- J. VET. MED., SER. B, Bd. 33, 1986, Seiten 321-339, XP002039313 MAYR A. ET AL.: "VERGLEICHENDE UNTERSUCHUNGEN ÜBER DIE IMMUNSTIMULIERENDE (PARAMUNISIERENDE) WIRKSAMKEIT VON BCG, LEVAMISOL, CORYNEBACTERIUM PARVUM UND PRÄPARATEN AUS POCKENVIREN IN VERSCHIEDENEN "IN VIVO"- UND "IN VITRO"-TESTEN"
- KLEINTIERPRAXIS, Bd. 37, 1992, Seiten 515-518, XP002039314 MAYR B. UND HÖRBER D.: "PARAMUNISIERUNG FeLV-POSITIVER KATZEN - EIN BERICHT AUS DER PRAXIS"
- STRAHLENTHERAPIE, Bd. 161, 1985, Seiten 168-176, XP002039315 BREITER N. ET AL: "UNTERSUCHUNGEN ÜBER DIE WIRKSAMKEIT DER PARAMUNITÄTSINDUCER PIND-AVI UND PIND-ORF ALS STRAHLENSCHUTZSUBSTANZEN"

## Beschreibung

Die vorliegende Erfindung betrifft die Verwendung von multipotenten Paramunitätsinducern (Pockeninducern) auf der Basis von Pocken- und Parapockenviren und deren Komponenten, einzeln oder in Kombination zur Herstellung von Arzneimitteln für neue Indikationsbereiche und Therapien, für die es bisher keine wirksamen und vor allem keine unschädlichen Arzneimittel gibt.

Präparate aus attenuierten, nicht vermehrungsfähigen und nicht immunogenen Pocken- und Parapockenviren (auch als "Pockeninducer" bezeichnet) greifen in den unspezifischen bzw. paraspezifischen, phylogentisch älteren Teil des komplexen Immunsystems (natural immune system, innate immune System, primitive immune System) bevorzugt regulierend, reparierend und restaurierend ein. Deshalb werden diese Pockeninducer nachstehend auch als "Bioregulantien" bezeichnet. Auf der zellulären Ebene aktivieren die Pockeninducer die Makrophagen, modulieren die NK-Zellen und weitere lymphoretikuläre Zellen, auf der humoralen Ebene steuern sie die Bildung verschiedener Zytokine, z.B. von Interleukin 1 α, Interleukin 2, 6 und 12, Tumornekrosefaktor, CSA oder von Interferonen (cytokine release). Eine Einführung in die Behandlung von Tieren mit Pockeninducern und eine Zusammenstellung der entsprechenden experimentellen Ergebnisse findet sich bei B. Mayr und A. Mayr: "Zum derzeitigen Stand der präklinischen Forschung über die Wirksamkeit und Unschädlichkeit von Paramunitätsinducern aus Pockenviren. Eine Literaturstudie", Tierärztl. Praxis, Bd. 23 (1995) 542-552. Dieses neue Prophylaxe- und Therapiekonzept hat sich in der Tiermedizin bewährt.

Wichtig und neu ist im Rahmen der vorliegenden Erfindung die überraschende Beobachtung, daß die durch Pockeninducer freigesetzten Zytokine die Bildung von Subtyp 1 der T-Helfer-zellen (Th1) begünstigen, wodurch - wie aus der Literatur bekannt - die Bildung von Subtyp 2 (Th2) blockiert wird. Th 2-Zellen steuern nämlich die humorale Immunantwort, die über Mastzellen zur Produktion von IgE allergische Reaktionen vom Soforttyp auslösen kann (antikörperbedingte Allergie). Die Th 1-Zellen können somit erfindungsgemäß als Antagonisten der Th 2-Zellen sofortreagierende Allergien verhindern bzw. bestehende Allergien vom Soforttyp abmildern. Die Th 1-Zellen agieren mit den zellulären erregerunspezifischen wie -spezifischen Zellen des Immunsystems und stehen in Verbindung (interagieren) mit den Zytokinen Interleukin 1 und 2, Interferonen, CSF und TNF, d.h. mit Zytokinen, die durch die Behandlung mit den Pockeninducern freigesetzt sind. Die Gefahr einer Auslösung von IgE bedingten Allergien ist damit nicht gegeben.

Pockeninducer haben sich inzwischen in der Humanmedizin wie in der Tiermedizin bezüglich Wirksamkeit und Unschädlichkeit vielfach bewährt. Die Verfahren zu ihrer Herstellung und ihre Anwendung als Arzneimittel zur Prophylaxe und Therapie bestimmter Krankheiten sind in der DE-C-44 05 841 belegt.

Die Herstellung der Pockeninducer erfolgt gemäß den in der DE-C-44 05 841 beschriebenen Verfahren. Die für die Produktion von Pockeninducern benötigten Ausgangsviren werden üblicherweise durch Vermehrung der attenuierten Pocken- und Parapockenvirusstämme in Zellkulturen, bevorzugt in der Zellinie VERO bzw. in primären oder sekundären Hühnerembryofibroblasten-Zellkulturen gewonnen. Verwendet werden nur Virusernten mit einem Infektiositätstiter von >10^{7,0} KID₅₀/ml. Die Virusernten der attenuierten Pockenstämme werden chemisch-pysikalisch, z.B. mit mindestens 0.05% β-Propiolacton bezogen auf den Pockenstamm inaktiviert. Nach einer Reinigung durch Zentrifugation bei 4000 g über 30 Minuten (oder andere chemisch-physikalische Methoden) werden dem Pockeninducer ein Stabilisator, wie ein Kohlenhydrat, ein mehrwertiger Zucker, ein Protein, bevorzugt Gelatine (Polygeline (Wz)) in einer Menge von 2.5%, bezogen auf den Pockenstamm zugesetzt. Das so gewonnene Präparat wird portioniert, lyophilisiert und bei +4 °C gelagert. Die Herstellung des gebrauchsfertigen Arzneimittels erfolgt, wie in der DE-C-44 05 841 beschrieben, durch Auflösung des Lyophilisates in sterilem Aqua dest. Eine Dosis entspricht 1 ml gelöstem Lyophilisat und soll mindestens 640, vorzugsweise mindestens 1280 VSV-Wirkungseinheiten enthalten.

Wie in der DE-C-44 05 841 beschrieben, können neben der Verwendung ganzer Viruspartikel auch Strukturelemente der einzelnen Virusstämme, z.B. Fusionsproteine, Adsorptionsproteine oder das 39 KD Protein von Parapockenviren, wie auch sog. "leere Hüllen" von Pockenviren für die Herstellung der Pockeninducer verwendet werden. Eine Inaktivierung ist in derartigen Fällen nicht mehr nötig.

Eine detaillierte Beschreibung der einzelnen Herstellungssschritte und -möglichkeiten von Pockeninducem aus Kombinationen mehrerer Pocken- bzw. Parapocken-Virusstämme findet sich in der DE-C-44 05 841.

Im Rahmen der Anwendung als Arzneimittel konnten nicht nur die in dieser Patentschrift aufgeführten Indikationen für die erfindungsgemäßen Pockeninducer aus Kombinationen von Pockenviren bestätigt werden, sondern es wurden überraschend vollkommen neue Anwendungsmöglichkeiten der Pockeninducer in bezug auf Prophylaxe und Therapie entdeckt, für die bislang keine wirksamen, parenteral oder oral zu verabreichenden und zugleich nachweislich unschädlichen Arzneimittel auch bei Dauerapplikation bzw. Überdosierung zur Verfügung stehen.

Dementsprechend betrifft die vorliegende Erfindung in Erweiterung der Lehre der DE-C-44 05 841 neue Indikationen für die Anwendung der Pockeninducer auf der Basis von Pocken- und Parapockenviren in der Therapie von Erkrankungen des Menschen. Diese neuen Indikationen beruhen auf dem überraschenden Befund, daß diese Pockeninducer ein gestörtes Immunsystem nicht nur im Sinne einer Erhöhung bzw. Stimulierung verminderter bzw. supprimierter Aktivitäten, sondern auch im Sinne einer Erniedrigung pathologisch erhöhter Immunparameter in den optimalen physiologischen Bereich_regulierend beeinflussen. Zum Verständnis der regulatorischen Wirkung von Pockeninducern im Sirme einer Bioregulation, die im Verbund zwischen Immun-, Hormon-und Nervensystem stattfindet, muß realisiert werden, daß sich die einzelnen humoralen wie zellulären Aktivitäten des Immunsystems gegenseitig stimulierend wie supprimierend beeinflussen und steuern. Das heißt, durch die Induktion zellulärer wie humoraler (löslicher) Komponenten des komplexen Immunsystems mit Pockeninducern entsteht ein sich selbst regulierendes System im Organismus. Dies kann z.B. dadurch zustandekommen, daß die Zahl oder Aktivität spezifischer Effektorzellen (z.B. Makrophagen, Leukozyten, T-Zellen, NK-Zellen) ansteigt bzw. sinkt oder die Produktion zellulärer und humoraler Kommunikationsmoleküle (z.B. IL 1 oder 2, Interferone etc.) zuoder abnimmt. Dabei reagiert jeder Organismus individuell je nach Funktionszustand seines Abwehrsystems zu Beginn der Behandlung mit dem Pockeninducer anders. Der hier verwendete Begriff "Bioregulation" durch Pockeninducer geht damit weit über das übliche Verständnis einer sog. "Immuntherapie" hinaus. So kann z.B. nach Pockeninducer-Behandlung bei einem Patienten die Zahl der Leukozyten ansteigen und umgekehrt die Zahl der NK-Zellen in ihrer Aktivität gleich bleiben oder sich sogar vermindern. Zum Ausgleich von Dysregulationen des Immunsystems muß es deshalb nicht gleichzeitig bei allen immunologischen Parametern zu einer Stimulierung oder Supprimierung kommen, weil lediglich bestehende Defizite bzw. überhöhte Werte ausgeglichen werden. Die Individualisierung in den physiologischen Normbereich durch Behandlung mit Pockeninducern ist deshalb eine ganz neue Erkenntnis. Da sich eine Intervention von Pockeninducern im individuellen Abwehrsystem gleichzeitig auch regulierend auf das Nerven- und Hormonsystem auswirkt, sollten Pockeninducer nicht als Immunregulantien definiert werden, sondern es wird hier vorgeschlagen, sie als Bioregulantien anzusehen. Ein derartiges Bioregulans, das auch bei starker Überdosierung unschädlich ist, die physiologischen "Normalwerte" des Immunsystems nicht beeinflußt und bei Dysfunktionen regulierend im Sinne einer physiologischen Normalisierung wirkt, also in seiner gesamten Funktionalität stabilisiert bzw. wiederhergestellt, gibt es bisher in der Medizin nicht. Pockeninducer eignen sich deshalb hervorragend zur prophylaktischen wie metaphylaktischen Anwendung bei Patienten mit einem dysregulierten Immunsystem, z.B. bei Immundefekten unterschiedlicher Genese, wie z.B. nach einer immunsuppressiven Therapie, bei immunsuppressiven Grundleiden oder durch exogene Einflüsse, wie Streßsituationen, außergewöhnliche Belastungen, lange Reisen, akutes Infektionsgeschehen in der Umgebung. Für die prophylaktische Paramunisierung genügen in der Regel 2 bis 3 Injektionen eines Pockeninducerpräparates an aufeinanderfolgenden Tagen kurz vor einer zu erwartenden Belastung. Metaphylaktische oder therapeutische Gaben von Pockeninducern sollten mindestens 3 Tage bzw, bis zum deutlichen Abklingen klinischer Symptome erfolgen. Nach schwereren Erkrankungen, vor allem nach dem Überstehen von Infektionskrankheiten, sollte die Rekonvaleszenz durch 2 bis 3 Injektionen eines Pockeninducerpräparates pro Woche bis zur völligen Wiederherstellung unterstützt werden.

Eine überraschende Anwendungsmöglichkeit der Pockeninducer einzeln oder in Kombination ist ihr Einsatz bei der praefinalen Tumortherapie, wie inoperabler und nicht mehr therapierbarer Tumorkrankheiten, zur Verminderung von unerträglichen Schmerzen und zur Verbesserung des Wohlbefindens und damit der Lebensqualität der Patienten. In derartigen Fällen werden die Pockeninducer mindestens 2 mal pro Woche bzw. nach dem subjektiven Empfinden des Patienten auch täglich verabreicht. Diese Behandlung ist kompatibel mit den derzeit verwendeten starken Analgetika wie Morphin. Sie führt sogar zu einer Verminderung bis Absetzung der vorher applizierten Analgetika.

Die erfindungsgemäß verwendeten Pockeninducer auf der Basis von Pocken- und Parapockenviren für die neuen Indikationen sind sowohl bei Überdosierung als auch bei Langzeitbehandlung gut verträglich und unschädlich. Ein parenteral applizierbares Therapeutikum, das diese Kriterien erfüllt, ist bisher nicht bekannt.

Die Unschädlichkeit einer Behandlung gilt in der Medizin als wichtigstes Kriterium. Bis heute gibt es auch noch kein Prophylaktikum bzw. Therapeutikurn, das für die genannten erfindungsgemäßen Indikationsgebiete eine ausreichende Wirksamkeit besitzt.

Die derzeit verwendeten bekannten sogenannten Immunmodulatoren, wie BCG, Levamisol, C. parvum und Freund'sches Adjuvans, sind erstens nicht unschädlich und wirken stets nur eingleisig, d.h. entweder stimulierend (Stimulantien, Adjuvantien u.a.) oder supprimierend (Immunsuppression). Ein Arzneimittel, das beide Eigenschaften besitzt und dabei im Sinne der hier definierten Bioregulation optimale physiologische Parameter erzielt, gibt es bisher nicht. Für die erfindungsgemäßen klinischen Anwendungsgebiete bzw. Indikationen, wie Schmerzbekämpfung im praefinalen Stadium, sind vergleichbare unschädliche und wirksarne Arzneimittel bisher nicht bekannt.

Die Applikation der erfindungsgemäß verwendeten Pockeninducer für die hier beschriebenen Indikationen kann parenteral oder lokal erfolgen, z.B. intranasal, als Aerosolspray, oral, rektal oder vaginal. Bevorzugt werden die Pockeninducer intramuskulär appliziert. Die Herstellung von Arzneipräparaten erfolgt in üblicher Weise. Die Präparate müssen aber auf einen pH-Wert von etwa 7 bis 8 eingestellt werden.

Die folgenden Beispiele dienen zur weiteren Erläuterung der Erfindung.

Als Pockeninducer werden hier Kombinationspräparate auf der Basis von Pocken- bzw. Parapockenviren beschrieben, die nachstehend als "Conpind" (Wz. angemeldet) bezeichnet werden. Gehalt pro Dosis 1280 VSV-Wirkungseinheiten. Eine Dosis wird in 1 ml Aqua dest aufgelöst.

### Beispiel 1

In einer immunologischen Arztpraxis wurden 15 Patienten mit unterschiedlichen Tumoren nach dem gleichen Schema wie in Beispiel 1 (6 Conpind-Dosen im Abstand von 3 bis 5 Tagen) behandelt. 4 Patienten kamen nach erfolgreicher Operation und abgeschlossener Bestrahlung, 3 nach der operativen Entfernung des Tumors und 8 nach einer Chemotherapie zur Behandlung in die Praxis. Ihr immunologischer Status wurde vor Beginn der Behandlungen sowie nach der letzten Behandlung untersucht. Alle Patienten wurden mindestens 1 Jahr beobachtet. Die Behandlungen wurden von allen Patienten sehr gut vertragen. Es wurden keine lokalen oder systemischen Nebenreaktionen beobachtet. Das Allgemeinbefinden besserte sich bei allen Patienten, außerdem hatte die Conpind-Behandlung einen positiven Einfluß auf Appetit und Stimmung der Patienten. Die Tumoren verkleinerten sich bei einigen Patienten. Metastasen traten im Beobachtungszeitraum von 1 Jahr nicht auf.

In den Tabellen III bis V ist an einzelnen Beispielen der Einfluß der Behandlung der Turmorpatienten mit Conpind auf immunologische Parameter zusammengestellt. Werte, die zu Beginn der Behandlung bereits im Normbereich lagen und sich nicht veränderten, sind nicht aufgeführt. Überraschend ist dabei, daß sich auch bei diesen Patienten einzelne Parameter erhöhten, während andere absanken. Häufig lagen diese Bewegungen zwar im Normbereich, zeigten aber doch deutlich an, daß der Patient auf die Behandlung positiv reagiert hatte. Interessant ist auch, daß selbst sehr alte Patienten wie die Patientin Nr. 27 mit 93 Jahren auf die Behandlung positiv reagiert hatten. Welche Funktionsmechanismen den gegenseitigen Reaktionen zwischen den einzelnen Parametern zugrunde liegen, ist noch unbekannt. Nachgewiesen ist jedoch, daß sich die einzelnen Parameter unterschiedlich nach einer Conpind-Behandlung in Richtung physiologischer Normalisierung verändern und diese Bioregulation stets zum gesundheitlichen Nutzen des Patienten führt.

**Tabelle III:**

| **Bioregulative Wirkung von Conpind auf den Immunstatus von Tumorpatienten nach Operation und Bestrahlung** | | | | | | |
|---|---|---|---|---|---|---|
| Patient | Indikation | Einfluß von Conpind auf immunologische Parameter | | | | Bewertung |
| | | Art | Normbereich | vor CP | nach CP | |
| Pat. 24 (m.) 58 Jahre | Colon-Ca., | Leukoz. | 4 000- 10000 | 3800 | 4600 | ↑ |
| | strahlen- | Th-Zellen | 29-59% | 29% | 35% | ↑ |
| | induzierte | Ts-Zellen | 19-48% | 33% | 43% | ↑ |
| | Immunsuppr | NK-Zellen | 6-29% | 42% | 32% | ↓ |
| Pat. 21 (m.) 43 Jahre | metastasier. | Leukoz. | 4000-10000 | 3800 | 9900 | ↑ |
| | Parotis-Ca. | NK-Zellen | 6-29% | 34% | 9% | ↓ |
| | | IL-2/CD4-Z. | 15-40% | 22% | 31% | ↑ |
| Pat. 30 (w.) 54 Jahre | Mamma-Ca. | Leukoz. | 4000-10000 | 4200 | 5500 | ↑ |
| | | CD4/CD8-R. | 0.8-2.8 | 2.3 | 2.6 | ↑ |
| | | Th-Zellen | 29-59% | 51% | 57% | ↑ |
| | | akt.T-Zellen | 0-12% | 13% | 7% | ↓ |
| | | IL-2/CD4-Z. | 15-40% | 38% | 15% | ↓ |

**Tabelle IV:**

| **Bioregulative Wirkung von Conpind auf den Immunstatus von Tumorpatienten nach der operativen Entfernung des Tumors** | | | | | | |
|---|---|---|---|---|---|---|
| Patient | Indikation | Einfluß von Conpind auf den immunolog.Status | | | | Bewertung |
| | | Art | Normbereich | vor CP | nach CP | |
| Pat. 22 (m.) 58 Jahre | Colon-Ca. | Leukoz. | 4000-10000 | 4300 | 7800 | ↑ |
| | | T-Zellen | 60-85% | 55% | 65% | ↑ |
| | | CD4/CD8-R. | 0.8-2.8 | 0.8 | 1.4 | ↑ |
| | | Th-Zellen | 29-59% | 35% | 49% | ↑ |
| | | Ts-Zellen | 19-48% | 46% | 35% | ↓ |
| | | NK-Zellen | 6-29% | 38% | 26% | |
| Pat. 28 (w.) 58 Jahre | Colon-Ca. | Leukoz. | 4000-10000 | 3600 | 5200 | ↑ |
| | | Th/Ts-Ratio | 0.8-2.8 | 0.8 | 1.9 | ↑ |
| | | Ts-Zellen | 19-48% | 42% | 33% | ↓ |

**Tabelle V:**

| **Bioregulative Wirkung von Conpind auf den Immunstatus von Tumorpatienten nach einer Chemotherapie** | | | | | | |
|---|---|---|---|---|---|---|
| Patient | Indikation | Einfluß von Conpind auf immunologische Parameter | | | | Bewertung |
| | | Art | Normbereich | vor CP | nach CP | |
| Pat. 19 (w.) 59 Jahre | Lungen-Ca. | Lymphoz. | 20-30% | 8% | 11% | ↑ |
| | | akt.T-Zellen | 0-12% | 17% | 12% | ↓ |
| | | NK-Zellen | 6-29% | 8% | 32% | ↑ |
| | | IL-2/CD4-Z. | 15-40% | 60% | 28% | ↓ |
| Pat. 25 (w.) 44 Jahre | malignes Melanom | Leukoz. | 4000-10000 | 9400 | 6700 | ↓ |
| | | Lymphoz. | 20-30% | 16% | 24% | ↑ |
| | | CD4/CD8-R. | 0.8-2.8 | 2.4 | 1.8 | ↓ |
| | | Th-Zellen | 29-59% | 48% | 53% | ↑ |
| | | Ts-Zellen | 19-48% | 20% | 26% | |
| | | ↓ NK-Zellen | 6-29% | 15% | 11% | ↑ |
| | | IL-2/CD4-Z. | 15-40% | 20% | 24% | |
| Pat. 27 (w.) 93 Jahre | rezidivier. Basaliom | Leuko | 4000-10000 | 6400 | 8900 | ↑ |
| | | T-Zellen | 60-85% | 90% | 80% | ↓ |
| | | CD4/CD8-R. | 0.8-2.8 | 0.8 | 1.4 | ↑ |
| | | IL-2/CD4-Z. | 15-40% | 12% | 21% | ↑ |

### Beispiel 2

Der Einfluß einer kontinuierlichen Behandlung mit Conpind auf die Entwicklung schädlicher Nebenreaktionen während bzw. nach einer Strahlen- bzw. Chemotherapie ist an 7 Tumorpatienten untersucht worden. 4 dieser Patienten war der Primärtumor operativ entfernt worden, 3 Patienten wurden wegen vorhandener Metastasen nicht operiert. Alle 7 Patienten erhielten eine Chemotherapie z.B. mit Cisplatin oder Endoxan. Zusätzlich unterzogen sich 2 operierte Patienten und 1 Patient ohne Operation einer Strahlentherapie. Sämtliche Patienten erhielten von Beginn der Strahlen- bzw. Chemotherapie täglich 1 Dosis Conpind sowie in den Behandlungsintervallen 2 bis 3 mal pro Woche je 1 Dosis Conpind. Bei keinem der Patienten (2 männlich, 5 weiblich) kam es zu den gefürchteten Chemotherapie- bzw. Strahlenschäden. Die Patienten fühlten sich wohl und positiv motiviert. Die Behandlung mit Conpind wurde nach Beendigung der Chemotherapie bzw. Strahlenbehandlung fortgesetzt: die folgenden 5 Wochen pro Woche an 2 Tagen je 1 Dosis Conpind, anschließend pro Monat 2 Dosen Conpind intramuskulär. Bis jetzt erfreuen sich die Conpind-Patienten bester Gesundheit (Beginn der Paramunisierung vor 3½ bzw. 2 Jahren). In retrospektiven Studien erlitten vergleichbare Patienten während bzw. nach einer Chemotherapie oder Strahlenbehandlung die bekannten schweren organischen und psychischen Schäden.

### Beispiel 3

Bei 5 Patienten mit inoperablen und austherapierten Tumorkrankheiten, die trotz entsprechender Schmerzbekämpfung unter stärksten Schmerzen und Depressionen litten und deshalb suizidgefährdet waren, wurden die Möglichkeiten der Behandlung mit Conpind zur Beeinflussung der Schmerzen und Behebung der Depressionen eingesetzt. Es handelte sich um 3 Patienten mit einem Kolonkarzinom, das bereits systemisch über den ganzen Körper metastasiert hatte. 2 Patientinnen litten an einem Mammakarzinom mit Metastasen im Gehirn, Rückenmark, Lunge und anderen Organen. Der kurz bevorstehende Tod waren bei allen 5 Patienten abzusehen. In diesem Stadium erhielten die Patienten täglich bzw. alle 2 bis 5 Tage bis zu ihrem Tod 1 Dosis Conpind intramuskulär. Bereits nach 3 bis 5 Applikationen nahmen die Schmerzen ab, teilweise verschwanden sie völlig. Auffällig und völlig unerwartet veränderte sich die Psyche der Patienten. Sie verloren ihre Depressionen, wurden bezüglich Gesundung optimistisch und fühlten sich subjektiv wohl. Zwei dieser Patienten verlangten sogar, aus der Klinik entlassen zu werden, da sie von ihrer Heilung überzeugt waren. Alle 5 Patienten starben jedoch innerhalb von 2 bis 5 Monaten nach Beginn der Conpind-Behandlung im Zustand eines optimistischen Wohlbefindens.

Bei einem weiteren Patienten mit generalisierendem Prostatakarzinom, der mit Morphin behandelt werden mußte, konnte bei Behandlung mit Conpind sofort Morphin ohne nachteilige Folgen abgesetzt werden.

Abschließende Bewertung der Unschädlichkeit der Pockeninducer, einzeln und in Kombination.

Die Unschädlichkeit der Pockeninducer PIND-AVI und PIND-ORF und der Kombination Conpind beweisen Langzeitbehandlungen im Selbstversuch bzw. im breiten Familien- und Freundeskreis. Von 1972 bis 1982 wurden Paramunitätsinducer aus Pockenviren (Vorläuferpräparate von Conpind, z.B. Duphapind® , Baypamun P® , PIND-AVI, PIND-ORF, Paravi) regelmäßig, d.h. mindestens einmal innerhalb von 2 Monaten, zur Behebung einer Infektanfälligkeit und zur Streßprophylaxe lokal (oral (gurgeln) oder nasal (aufschnupfen)) verwendet. Eine lokale Kur bestand in 6 bis 10 Applikationen im Abstand von 4 bis 24 Stunden.

Weitere Personen aus dem Familien- und Freundeskreis unterzogen sich ab 1983 regelmäßigen parenteralen Behandlungen mit Pockeninducern. Es wurden dabei pro Jahr 6 bis 8 Kuren mit jeweils 2 bis 3 Injektionen von je 1 Dosis Pockeninducer ad us. vet. (kein Kombinationspräparat, z.B. Duphapind® , Baypamun P® ) zur Prophylaxe und Metaphylaxe durchgeführt.

Seit 1993 verwendet der gleiche Personenkreis zu diesem Zweck Conpind, wobei bei akuter Infektionsgefahr häufig auch die lokale und parenterale Applikation kombiniert werden. In keinem der genannten Fälle sind bisher negative Nebenreaktionen oder Allergien aufgetreten, während die Wirksamkeit voll erhalten blieb. Neben der parenteralen, oralen und nasalen Anwendung hat sich dabei bei schlecht heilenden Wunden oder Ulcera auch die lokale Applikation mittels Salben bewährt.

## Patentansprüche

1. Verwendung von Pockeninducern aus Kombinationen mehrerer Pocken- bzw. Parapocken-Virusstämme zur Herstellung von Arzneimitteln zur präfinalen Tumortherapie zur Verminderung von Schmerzen und Optimierung des Wohlbefindens der Patienten.

2. Verwendung gemäß Anspruch 1, zur Herstellung eines Arzneimittels zur parenteralen oder lokalen Verabreichung.

3. Verwendung gemäß einem der Ansprüche 1 oder 2, zur Herstellung eines Arzneimittels zur intranasalen, oralen, rektalen, vaginalen oder intramuskulären Verabreichung.

4. Verwendung gemäß einem der Ansprüche 1 bis 3, zur Herstellung eines Arzneimittels zur zweimal wöchentlichen Verabreichung.

## Claims

1. Use of pox inducers of combinations of several poxvirus and parapoxvirus strains, respectively, for the preparation of medicaments for prefinal tumor therapy to relief pain and optimize well-being of patients.

2. Use of claim 1 for the preparation of a medicament for parenteral or local administration.

3. Use of any one of claim 1 or 2 for the preparation of a medicament for intranasal, oral, rectal, vaginal or intramuscular administration.

4. Use of any one of claims 1-3 for the preparation of a medicament for twice weekly administration.

## Revendications

1. Utilisation d'inducteurs de pox obtenus à partir de combinaisons de plusieurs souches respectivement de pox- et de parapox-virus pour la préparation de médicaments pour la thérapie tumorale préfinale pour l'atténuation des douleurs et l'optimisation du bien-être des patients.

2. Utilisation selon la revendication 1, pour la préparation d'un médicament pour administration parentérale ou locale.

3. Utilisation selon l'une des revendications 1 ou 2, pour la préparation d'un médicament pour administration intranasale, orale, rectale, vaginale ou intramusculaire.

4. Utilisation selon l'une des revendications 1 à 3, pour la préparation d'un médicament pour administration deux fois par semaine.
